(19) Europäisches Patentamt
European Patent Office
Office européen des brevets

(11) **EP 3 022 578 B1**

(12) **FASCICULE DE BREVET EUROPEEN**

(45) Date de publication et mention
de la délivrance du brevet:
**04.10.2017 Bulletin 2017/40**

(21) Numéro de dépôt: **14750574.7**

(22) Date de dépôt: **16.07.2014**

(51) Int Cl.:
*G01S 7/52* *(2006.01)*    *G01S 15/89* *(2006.01)*

(86) Numéro de dépôt international:
**PCT/FR2014/051829**

(87) Numéro de publication internationale:
**WO 2015/007992 (22.01.2015 Gazette 2015/03)**

(54) **PROCÉDÉ ET DISPOSITIF DE CARTOGRAPHIE DE MILIEUX FIBREUX**

VERFAHREN UND VORRICHTUNG ZUR ABBILDUNG VON FASERMEDIEN

METHOD AND DEVICE FOR MAPPING FIBROUS MEDIA

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **19.07.2013 FR 1357158**

(43) Date de publication de la demande:
**25.05.2016 Bulletin 2016/21**

(73) Titulaire: **Centre National de la Recherche
Scientifique
(C.N.R.S.)
75794 Paris Cedex 16 (FR)**

(72) Inventeurs:
• **TANTER, Mickaël**
  **F-92220 Bagneux (FR)**
• **FINK, Mathias**
  **F-92190 Meudon (FR)**
• **PERNOT, Mathieu**
  **F-75004 Paris (FR)**
• **PAPADACCI, Clément**
  **F-75015 Paris (FR)**

(74) Mandataire: **Cabinet Plasseraud
66, rue de la Chaussée d'Antin
75440 Paris Cedex 09 (FR)**

(56) Documents cités:
**EP-A2- 2 101 191    US-A1- 2013 109 971**

• **WEI-NING LEE ET AL: "Noninvasive assessment
of myocardial anisotropy in vitro and in vivo using
Supersonic Shear Wave Imaging",
ULTRASONICS SYMPOSIUM (IUS), 2010 IEEE,
IEEE, 11 octobre 2010 (2010-10-11), pages
690-693, XP031953033, DOI:
10.1109/ULTSYM.2010.5935898 ISBN:
978-1-4577-0382-9**
• **MONTALDO G ET AL: "Coherent plane-wave
compounding for very high frame rate
ultrasonography and transient elastography",
IEEE TRANSACTIONS ON ULTRASONICS,
FERROELECTRICS AND FREQUENCY
CONTROL, IEEE, US, vol. 56, no. 3, 1 mars 2009
(2009-03-01), pages 489-506, XP011255897, ISSN:
0885-3010 cité dans la demande**
• **BASTIEN DENARIE ET AL: "Coherent Plane Wave
Compounding for Very High Frame Rate
Ultrasonography of Rapidly Moving Targets",
IEEE TRANSACTIONS ON MEDICAL IMAGING,
IEEE SERVICE CENTER, PISCATAWAY, NJ, US,
vol. 32, no. 7, 1 juillet 2013 (2013-07-01), pages
1265-1276, XP011516355, ISSN: 0278-0062, DOI:
10.1109/TMI.2013.2255310 cité dans la demande**

**Description**

DOMAINE DE L'INVENTION

La présente invention est relative aux procédés et dispositifs de cartographie de milieux fibreux.

ARRIERE-PLAN DE L'INVENTION

Un tel procédé a déjà été décrit par exemple par Derode et Fink (Spatial coherence of ultrasonic speckle in composites, Derode A., Fink M., IEEE Trans. Ultrason. Ferroelectr. Freq. Control, 1993;40(6):666-75), qui enseigne de faire émettre successivement des ondes ultrasonores focalisées par une barrette de transducteur disposée à la surface d'un matériau composite, avec plusieurs orientations de la barrette de transducteurs. On calcule pour chaque tir d'onde ultrasonore, une fonction de cohérence spatiale entre les signaux captés par les transducteurs suite à la réverbération de l'onde ultrasonore émise, et on détermine la direction des fibres du matériau composite comme étant la direction de la barrette de transducteurs correspondant au maximum de la fonction de cohérence spatiale.

Ce procédé connu convient pour un milieu simple tel qu'un matériau composite dont les fibres sont disposées régulièrement ; il ne convient pas pour étudier un milieu plus complexe tel qu'un tissu biologique.

Or, il existe un besoin pour cartographier la structure des tissus biologiques composés de fibres tels que notamment le myocarde, les muscles et le cerveau. Cette structure joue un rôle crucial à la fois dans la fonction mécanique (tissus musculaires) mais aussi électrique (cerveau, muscle, coeur) de ces tissus, et l'orientation des fibres dans l'espace est donc un paramètre très important à déterminer pour le diagnostic et l'exploration fonctionnelle de ces organes.

Par exemple, en imagerie cérébrale, il est très important d'identifier les faisceaux de fibres neuronales qui relient entre eux les différentes zones cérébrales. Actuellement, la seule technique capable de fournir une image tridimensionnelle de l'organisation des fibres est l'Imagerie par Résonance Magnétique de tenseur de diffusion (IRM de diffusion). Cette technique très lente à mettre en oeuvre est utilisée pour l'exploration du cerveau adulte, mais reste trop limitée pour imager les organes en mouvements tels que le coeur. Enfin l'IRM n'est pas utilisée pour imager le cerveau de très jeunes enfants, en particulier les bébés prématurés qui peuvent présenter des anomalies du développement cérébral malheureusement impossible à diagnostiquer avec les techniques actuelles.

RESUME DE L'INVENTION

La présente invention a notamment pour but de pallier ces inconvénients.

A cet effet, l'invention propose un procédé de cartographie de milieu fibreux, comprenant :

(a) une étape de mesure au cours de laquelle on fait émettre par un réseau bidimensionnel de transducteurs $T_{ij}$, dans un champ d'observation appartenant à un milieu comportant des fibres, un nombre N d'ondes ultrasonores incidentes non focalisées (c'est-à-dire non focalisées dans le champ d'observation) $I$ ayant des fronts d'ondes différents, et on fait capter par les transducteurs $T_{ij}$ des signaux $RFraw_{I,ij}$ (t) représentatifs d'ondes ultrasonores réverbérées par le milieu respectivement à partir des ondes incidentes $I$,

(b) une étape de synthèse de données cohérentes au cours de laquelle on détermine à partir des N ensembles de signaux $RFraw_{I,ij}(t)$ captés, pour un nombre M de points de focalisation fictifs $P_k$ dans le champ d'observation, des signaux cohérents $RFcoherent_{k,ij}(t)$ correspondant aux signaux qui auraient été reçus par les transducteurs $T_{ij}$ si une onde focalisée au point $P_k$ avait été émise par lesdits transducteurs,

(c) une étape de cartographie des fibres du milieu, au cours de laquelle on détermine une présence et une orientation de fibres en chaque point $P_k$, en comparant une cohérence spatiale entre les signaux cohérents $RFcoherent_{k,ij}$ (t) selon plusieurs directions.

On peut ainsi, très rapidement et aisément, cartographier la structure des tissus biologiques composés de fibres tels que le myocarde et autres muscles, et le cerveau, grâce au fait que les signaux rétrodiffusés contiennent des informations sur la microstructure des tissus qui ne sont pas visible directement sur l'image échographique (B mode). C'est l'analyse de la cohérence spatiale qui révèle l'orientation des fibres car l'anisotropie des tissus se retrouve au niveau de la fonction de cohérence mesurée dans différentes directions.

Dans divers modes de réalisation du procédé selon l'invention, on peut éventuellement avoir recours en outre à l'une et/ou à l'autre des dispositions suivante :

- au cours de l'étape (c), on détermine une intégrale d'une fonction de cohérence spatiale entre transducteurs dans plusieurs directions, et on détermine la direction des fibres comme étant une direction maximisant ladite intégrale ;
- les ondes ultrasonores incidentes sont des ondes planes ayant différentes directions de propagation ;

- les ondes ultrasonores incidentes sont des ondes divergentes (émises par la barrette ultrasonore comme si elles provenaient de différents points sources) ;
- les ondes ultrasonores incidentes sont émises successivement ;
- les ondes ultrasonores incidentes sont codées spatio-temporellement et émises simultanément, puis les ondes réverbérées sont captées simultanément puis séparées par décodage ;
- on affiche une image des fibres détectées dans le milieu ;
- on détermine une image échographique du champ d'observation et on affiche cette image échographique en superposition avec l'image des fibres ;
- on détermine l'image échographique par formation de voies à partir des signaux cohérents déterminés à l'étape (b) ;
- le milieu à imager est un tissu humain ou animal (notamment mammifère).

Par ailleurs, l'invention a également pour objet un dispositif pour la mise en oeuvre d'un procédé de cartographie tel que défini ci-dessus, comprenant un réseau bidimensionnel de transducteurs $T_{ij}$ et des moyens de commande et traitement adaptés pour :

(a) faire émettre par le réseau bidimensionnel de transducteurs $T_{ij}$, dans un champ d'observation d'un milieu comportant des fibres, un nombre N d'ondes ultrasonores incidentes 1 ayant des fronts d'ondes différents, et faire capter par les transducteurs $T_{ij}$ des signaux $RFraw_{l,ij}$ (t) représentatifs d'ondes ultrasonores réverbérées respectivement à partir des ondes incidentes $l$,
(b) déterminer à partir des N ensembles de signaux $RFraw_{l,ij}$ (t) captés, pour un nombre M de points de focalisation fictifs $P_k$ dans le champ d'observation, des signaux cohérents $RFcoherent_{k,ij}(t)$ correspondant aux signaux qui auraient été reçus par les transducteurs $T_{ij}$ si une onde focalisée au point $P_k$ avait été émise par lesdits transducteurs,
(c) déterminer une présence et une orientation de fibres en chaque point $P_k$, en comparant des fonctions de cohérence spatiale entre les signaux $RFcoherent_{k,ij}(t)$ selon plusieurs directions.

BREVE DESCRIPTION DES DESSINS

D'autres caractéristiques et avantages de l'invention apparaîtront au cours de la description suivante d'un de ses modes de réalisation, donné à titre d'exemple non limitatif, en regard des dessins joints.
Sur les dessins :

- la figure 1 est une vue schématique d'un dispositif pour la mise en oeuvre d'un procédé selon un mode de réalisation de l'invention, et
- la figure 2 est un schéma bloc d'une partie du dispositif de la figure 1.

DESCRIPTION DETAILLEE

Sur les différentes figures, les mêmes références désignent des éléments identiques ou similaires.

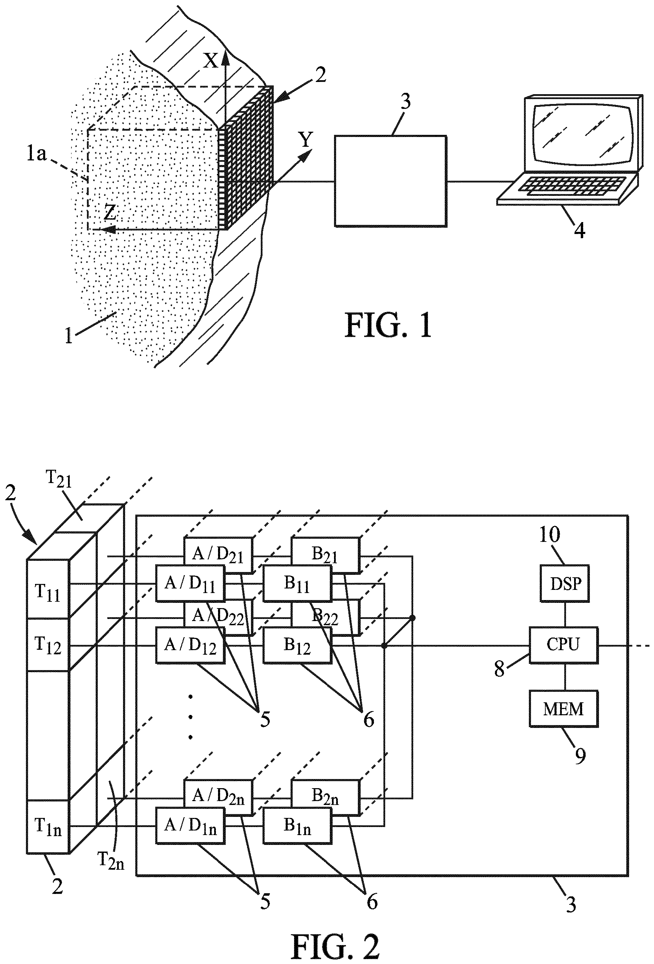

La figure 1 montre un exemple de dispositif d'imagerie fonctionnant par émission et réception d'ondes ultrasonores de compression, par exemple de fréquences comprises entre 2 et 40 MHz.
Le dispositif d'imagerie représenté sur la figure 1 est adapté pour réaliser une imagerie ultrasonore synthétique d'un champ d'observation 1a dans un milieu fibreux 1, par exemple des tissus d'un patient, notamment un muscle (myocarde ou autre) ou un cerveau.
Le dispositif d'imagerie comporte par exemple :

- un réseau 2 de n transducteurs ultrasonores, par exemple un réseau à deux dimensions comprenant par exemple quelques centaines de transducteurs et adapté pour réaliser une image tridimensionnelle (3D) du champ d'observation 1a ;
- une baie électronique 3 ou similaire commandant le réseau de transducteurs 2 et adaptée pour acquérir les signaux captés par ce réseau de transducteurs ;
- un ordinateur 4 ou similaire pour commander la baie électronique 3 et visualiser les images ultrasonores obtenues à partir desdits signaux captés.

Le réseau 2 de transducteurs peut par exemple être un réseau matriciel plan s'étendant selon deux axes perpendiculaires X, Y, l'axe Z perpendiculaire aux axes X, Y désignant la direction de la profondeur dans le champ d'observation. Dans ce qui suit, les transducteurs seront notés $T_{ij}$, i et j étant deux indices désignant le rang de chaque transducteur respectivement selon les axes X et Y. Le réseau 2 de transducteurs peut notamment comprendre $n_1$ transducteurs dans la direction X et $n_2$ transducteurs dans la direction Y, avec $n= n_1*n_2$. La description qui suit sera faite en prenant pour exemple ce type de réseau 2 de transducteurs, mais d'autres formes de de réseau de transducteurs sont également possibles dans le cadre de la présente invention.

Comme représenté sur la figure 2, la baie électronique 3 peut comprendre par exemple :

- n convertisseurs analogique / digital 5 (A/D$_{ij}$) connectés individuellement aux n transducteurs T$_{ij}$ du réseau 2 de transducteurs,
- n mémoires tampon 6 (B$_{ij}$) respectivement connectées aux n convertisseurs analogique / digital 5,
- une unité centrale 8 (CPU) communiquant avec les mémoires tampon 6 et l'ordinateur 4,
- une mémoire 9 (MEM) connectée à l'unité centrale 8,
- un processeur numérique de signal 10 (DSP) connecté à l'unité centrale 8.

Ce dispositif permet de mettre en oeuvre un procédé de cartographie des fibres du milieu 1, qui inclut notamment les trois étapes suivantes, mises en oeuvre par l'unité centrale 8 assistée du processeur numérique de signal 9 :

a) Mesure (émission / réception et enregistrement des données brutes),
b) Synthèse de données cohérentes,
c) Analyse de l'orientation des fibres,
d) Eventuellement, détermination d'une image du milieu en B mode et superposition avec la cartographie des fibres.

**Etape a: Mesure (émission / réception et enregistrement des données brutes) :**

Le réseau de transducteur est mis en contact avec le milieu 1 et un nombre N d'ondes incidentes ultrasonores est émis dans le milieu 1 par les transducteurs T$_{ij}$ (N peut être compris par exemple entre 2 et 100, notamment entre 5 et 10). Les ondes incidentes en question sont non focalisées (plus précisément, non focalisées dans le champ d'observation) et ont des respectivement fronts d'onde différents, c'est-à-dire des fronts d'onde de formes différentes et / ou d'orientation différentes. Avantageusement, les ondes incidentes peuvent être des ondes planes d'inclinaisons toutes différentes, caractérisées par leurs angles d'inclinaison respectifs $\alpha_X$, $\alpha_Y$ par rapport à l'axe Z, respectivement dans les plans (X, Z) et (Y, Z), ou encore des ondes divergentes émises comme si elles provenaient de différents points de l'espace.

Les ondes incidentes sont généralement des impulsions de moins d'une microseconde, typiquement environ 1 cycle de l'onde ultrasonore à la fréquence centrale. Les tirs d'ondes incidentes peuvent être séparés les uns des autres par exemple d'environ 50 à 200 microsecondes.

Chaque onde incidente rencontre dans le milieu 1 des diffuseurs qui réverbèrent l'onde incidente. L'onde ultrasonore réverbérée est captée par les transducteurs T$_{ij}$ du réseau. Le signal ainsi capté par chaque transducteur T$_{ij}$ provient de l'ensemble du milieu 1, puisque l'onde incidente n'est pas focalisée en émission.

Les signaux réverbérés captés par les n transducteurs T$_{ij}$ sont alors numérisées par les convertisseurs analogique-digital correspondants A/D$_{ij}$ est mémorisé dans les mémoires tampon correspondantes B$_{ij}$. Les signaux ainsi mémorisés dans les mémoires tampon après chaque tir incidents seront appelées ci-après données brutes RF ("RF" est un terme usuellement utilisé dans le domaine, simplement du fait de la fréquence des ultrasons utilisés). Ces données brutes RF consistent en une matrice de $n_1 * n_2$ signaux temporels RFraw$_{l,ij}$ (t) captés respectivement par les transducteurs T$_{ij}$ après le tir $l$ d'onde ultrasonore incidente.

Après chaque tir $l$ d'onde incidente, les signaux mémorisés dans les mémoires tampons B$_{ij}$ sont transférés dans la mémoire 9 du processeur de signal 8 aux fins de traitement par ce processeur. A la fin de l'étape (a), la mémoire 9 contient donc N matrices de signaux bruts RF.

On notera que les différentes ondes incidentes pourraient être codées spatio-temporellement, de façon à permettre une émission simultanée de tout ou partie des ondes incidentes et une réception également simultanée des ondes réverbérées, qui sont alors séparées par décodage avant mémorisation.

**Etape b: Synthèse de données cohérentes RF**

A partir des N matrices de données brutes RF, un nombre M de matrices de données cohérentes synthétiques RF est calculé par le processeur 8, respectivement en M points P$_k$(x, y, z) du champ d'observation 1a (k étant un entier compris entre 1 et M et x, y, z étant les coordonnées du point P$_k$ sur les axes X, Y, Z). Chacune de ces M matrices de données cohérentes synthétiques RF comporte $n_1 * n_2$ signaux temporels RFcoherent$_{k,ij}$ (t) correspondant aux signaux qui seraient captés respectivement par les transducteurs T$_{ij}$ si les transducteurs émettaient une onde incidente focalisée au point P$_k$.

Les matrices de données RF cohérentes peuvent être obtenues par exemple en supposant une vitesse de propagation c homogène dans tout le milieu 1 pour les ondes de compression ultrasonores, selon le principe expliqué notamment dans le document EP2101191 ou dans l'article de Montaldo et al. « Coherent plane-wave compounding for very high frame rate ultrasonography and transient elastography » (IEEE Trans Ultrason Ferroelectr Freq Control 2009 Mar ; 56(3): 489-506).

La direction de propagation de l'onde plane correspondant à chaque tir 1 étant connue, et la vitesse de propagation c étant connue, le processeur 8 peut calculer pour chaque point $P_k$ le temps de propagation $\tau_{ec}(l,k)$ de l'onde incidente *l* jusqu'au point $P_k$, et le temps de propagation $\tau_{rec}(l,k, i, j)$ de l'onde réverbérée depuis le point $P_k$ vers le transducteur $T_{ij}$, donc le temps de trajet total aller-retour $\tau(l,k, i, j) = \tau_{ec}(l,P_k) + \tau_{rec}(l,P_k, i, j)$.

Le signal spatialement cohérent pour le transducteur Tij, correspondant au point de focalisation virtuel $P_k$, est alors calculé selon la formule :

$$RFcoherent_{kij}(t) = \sum_l B(l) RFraw_{lij}(\tau(l,k,i,j)) \tag{1}$$

où B(*l*) est une fonction de pondération pour la contribution de chaque tir *l* d'onde incidente (dans les cas courants, les valeurs B(*l*) peuvent être toutes égales à 1).

Les matrices de données cohérentes RFcoherent$_k$ peuvent ensuite être éventuellement affinées en corrigeant les effets d'aberrations dans le milieu 1, par exemple comme expliqué dans les documents susmentionnés EP2101191 ou Montaldo et al.

## Etape c: Analyse de l'orientation des fibres

On détermine ensuite, pour chaque matrice RFcoherent$_k$, une cohérence spatiale, indicatrice de la cohérence entre les signaux RFcoherent-$_{kij}$(t-) pour un même point $P_k$.

Cette cohérence spatiale peut être mesurée par exemple par une fonction de cohérence spatiale R(m) calculée à partir des corrélations des signaux c$_k$(ij,pq) reçus sur des transducteurs ij et pq, en sommant toutes corrélations entre les paires de transducteurs distants de m éléments dans une direction donnée du plan (X,Y).

$$c(ij,tu) = \sum_{T1}^{T2} (RFcoherent_{k,ij}(t) - \overline{RFcoherent_{k,ij}})(RFcoherent_{k,tu}(t) - \overline{RFcoherent_{k,tu}})$$
$$(2)$$

Où $\overline{RFcoherent_{k,ij}}$ est une moyenne temporelle de RFcoherent$_{k,ij}$, et T1, T2 sont deux instants.

En considérant uniquement des transducteurs alignés entre eux selon une même direction du plan (X, Y) et en renumérotant ces transducteurs Tq, q allant de 1 à Q, ces corrélations peuvent s'écrire c(p, q) et on obtient :

$$c(p,q) = \sum_{T1}^{T2} (RFcoherent_{k,p}(t) - \overline{RFcoherent_{k,p}})(RFcoherent_{k,q}(t) - \overline{RFcoherent_{k,q}})$$
$$(2bis)$$

$$R(m) = \frac{Q}{Q-m} \frac{\sum_{q=1}^{Q-m} c(q,q+m)}{\sum_{q=1}^{Q} c(q,q)} \tag{3}$$

Le théorème de Van Cittert Zernike établit l'allure de cette fonction R(m) dans un milieu de diffuseur aléatoire (donc isotrope) pour un faisceau monochromatique. R(m) est la transformée de Fourier spatiale au carré de la tâche focale. Pour une tâche focale dont l'extension latérale est donnée par une fonction sin(ax)/x, R(m) est un triangle dont le sommet est en m=0 (autocorrélation) et qui s'annule en m=Q.

Pour un milieu non isotrope, un surcroit de cohérence spatiale est obtenu lorsque la direction d'alignement des transducteurs est alignée le long des fibres.

L'intégrale S$_k$ de cette fonction dans la direction d'alignement considérée du plan (X, Y) donne un paramètre de cohérence spatiale, qui est maximum dans la direction d'alignement des fibres. En calculant ce paramètre de cohérence spatiale dans plusieurs directions d'alignement des transducteurs, on peut connaître la direction produisant le paramètre de cohérence spatiale S$_k$ maximum et donc en déduire la direction des fibres au point $P_k$.

On notera que les fonctions de cohérence spatiales R(m) susmentionnées ou les paramètres de cohérence

spatiale $S_k$ pourraient être moyennées sur plusieurs points $P_k$ voisins, donc dans un petit volume du champ d'observation autour d'un point d'intérêt.

Un autre paramètre de cohérence spatiale possible est le critère de focalisation $C_k$ qui donne le ratio entre l'énergie cohérente et l'énergie incohérente rétrodiffusée. Avec les notations ci-dessus, c'est-à-dire en numérotant de q=1 à Q les transducteurs alignés selon une même direction du plan (X,Y), on a :

$$C_k = \frac{\left\langle \left| \sum_{q=1}^{Q} RFcoherent_{kq}\left(t-tq\right) \right|^2 \right\rangle}{Q\sum_{q=1}^{Q} \left\langle \left| RFcoherent_{kq}\left(t-tq\right) \right|^2 \right\rangle} \qquad (4)$$

Où $t_q$ est un retard permettant de remettre en phase tous les signaux RFcoherent$_{kq}$(t).

Comme dans le cas précédent, on calcule ce paramètre de cohérence spatiale selon plusieurs directions pour chaque point $P_k$, et on détermine la direction des fibres comme la direction maximisant le paramètre Ck.

On peut ainsi déterminer une cartographie tridimensionnelle des fibres du milieu dans le champ d'observation 1a, de façon très rapide. Cette cartographie peut avantageusement être présentée à l'utilisateur du dispositif sous la forme d'images de plans de coupe du milieu 1, affichées par exemple sur l'écran de l'ordinateur 4. Eventuellement, ces images peuvent être calculées en reconstituant une continuité entre les fibres détectées aux différents points $P_k$.

**Etape d: formation d'image**

A partir des matrices RFcoherent$_k$ calculées à l'étape (b), on peut éventuellement former une image tridimensionnelle B mode du champ d'observation 1a, par formation de voie en réception, comme expliqué par exemple dans le document EP2101191 susmentionné.

Cette image B mode peut être éventuellement superposée à la cartographie des fibres déterminée à l'étape (c), et on peut afficher sur l'écran de l'ordinateur des images de plans de coupe du champ d'observation montrant à la fois l'image B mode et les fibres superposées à cette image.

**Revendications**

1. Procédé de cartographie de milieu fibreux, comprenant :

   (a) une étape de mesure au cours de laquelle on fait émettre par un réseau bidimensionnel (2) de transducteurs $T_{ij}$, dans un champ d'observation (1a) appartenant à un milieu (1) comportant des fibres, un nombre N d'ondes ultrasonores incidentes $I$ non focalisées ayant des fronts d'ondes différents, et on fait capter par les transducteurs $T_{ij}$ des signaux RFraw$_{I,ij}$(t) représentatifs d'ondes ultrasonores réverbérées par le milieu respectivement à partir des ondes incidentes $I$,
   (b) une étape de synthèse de données cohérentes au cours de laquelle on détermine à partir des N ensembles de signaux RFraw$_{I,ij}$(t) captés, pour un nombre M de points de focalisation fictifs $P_k$ dans le champ d'observation, des signaux cohérents RFcoherent$_{k,ij}$ (t) correspondant aux signaux qui auraient été reçus par les transducteurs $T_{ij}$ si une onde focalisée au point $P_k$ avait été émise par lesdits transducteurs,
   (c) une étape de cartographie des fibres du milieu (1), au cours de laquelle on détermine une présence et une orientation de fibres en chaque point $P_k$, en comparant une cohérence spatiale des signaux cohérents RFcoherent$_{k,ij}$(t) selon plusieurs directions.

2. Procédé selon la revendication 1, dans lequel au cours de l'étape (c), on détermine une intégrale de fonctions de cohérence spatiale entre transducteurs dans plusieurs directions, et on détermine la direction des fibres comme étant une direction maximisant ladite intégrale.

3. Procédé selon l'une quelconque des revendications précédentes, dans lequel les ondes ultrasonores incidentes sont des ondes planes ayant différentes directions de propagation.

4. Procédé selon l'une quelconque des revendications 1 à 3, dans lequel les ondes ultrasonores incidentes sont des ondes divergentes.

**5.** Procédé selon l'une quelconque des revendications précédentes, dans lequel on affiche une image des fibres détectées dans le milieu (1).

**6.** Procédé selon la revendication 5, dans lequel on détermine une image échographique du champ d'observation (1a) et on affiche cette image échographique en superposition avec l'image des fibres.

**7.** Procédé selon la revendication 6, dans lequel on détermine l'image échographique par formation de voies à partir des signaux cohérents déterminés à l'étape (b).

**8.** Procédé selon l'une quelconque des revendications précédentes, dans lequel le milieu à imager est un tissu humain ou animal.

**9.** Dispositif pour la mise en oeuvre d'un procédé de cartographie selon l'une quelconque des revendications précédentes, comprenant un réseau bidimensionnel (2) de transducteurs $T_{ij}$ et des moyens de commande et traitement (8, 4) adaptés pour :

(a) faire émettre par le réseau bidimensionnel (2) de transducteurs $T_{ij}$, dans un champ d'observation (1a) d'un milieu (1) comportant des fibres, un nombre N d'ondes ultrasonores incidentes $I$ ayant des fronts d'ondes différents, et faire capter par les transducteurs $T_{ij}$ des signaux $RFraw_{l,ij}$ (t) représentatifs d'ondes ultrasonores réverbérées respectivement à partir des ondes incidentes $I$,

(b) déterminer à partir des N ensembles de signaux $RFraw_{l,ij}$ (t) captés, pour un nombre M de points de focalisation fictifs $P_k$ dans le champ d'observation, des signaux cohérents $RFcoherent_{k,ij}$ (t) correspondant aux signaux qui auraient été reçus par les transducteurs $T_{ij}$ si une onde focalisée au point $P_k$ avait été émise par lesdits transducteurs,

(c) déterminer une présence et une orientation de fibres en chaque point $P_k$, en comparant une cohérence spatiale entre les signaux cohérents $RFcoherent_{k,ij}$ (t) selon plusieurs directions.

**Patentansprüche**

**1.** Verfahren zur Abbildung von einem Fasermedium, umfassend:

(a) einen Schritt des Messens, bei dem von einem zweidimensionalen Array (2) von Wandlern $T_{ij}$ in einem Beobachtungsfeld (1a), das zu einem Medium (1) gehört, das Fasern aufweist, eine Anzahl N von nichtfokussierten, einfallenden Ultraschallwellen $I$, die unterschiedliche Wellenfronten aufweisen, emittiert wird, und von den Wandlern $T_{ij}$ für Ultraschallwellen repräsentative Signale $RFraw_{l,ij}$(t) einfangen werden, die von dem Medium jeweils ausgehend von den einfallenden Wellen $I$ zurückgestrahlt werden,

(b) einen Schritt der Synthese von kohärenten Daten, bei dem ausgehend von den N Sätzen von eingefangenen Signalen $RFraw_{l,ij}$(t) für eine Anzahl M von fiktiven Fokuspunkten $P_k$ in dem Beobachtungsfeld kohärente Signale $RFcoherent_{k,j}$(t) bestimmt werden, die den Signalen entsprechen, die von den Wandlern $T_{ij}$ empfangen worden wären, wenn eine an dem Punkt $P_k$ fokussierte Welle von den Wandlern emittiert worden wäre,

(c) einen Schritt der Abbildung der Fasern des Mediums (1), bei dem eine Anwesenheit und eine Ausrichtung von Fasern an jedem Punkt $P_k$ bestimmt wird, indem eine räumliche Kohärenz der kohärenten Signale $RFcoherent_{k,ij}$(t) in verschiedenen Richtungen verglichen wird.

**2.** Verfahren nach dem Anspruch 1, wobei bei dem Schritt (c) ein Integral von Funktionen von räumlicher Kohärenz zwischen Wandlern in mehreren Richtungen bestimmt wird und die Richtung der Fasern als eine Richtung bestimmt wird, die das Integral maximiert.

**3.** Verfahren nach einem der vorhergehenden Ansprüche, wobei die einfallenden Ultraschallwellen ebene Wellen sind, die verschiedene Ausbreitungsrichtungen aufweisen.

**4.** Verfahren nach einem der Ansprüche 1 bis 3, wobei die einfallenden Ultraschallwellen divergierende Wellen sind.

**5.** Verfahren nach einem der vorangehenden Ansprüche, wobei ein Bild der Fasern, die in dem Medium (1) erfasst werden, angezeigt wird.

**6.** Verfahren nach Anspruch 5, wobei ein Ultraschallbild des Beobachtungsfeldes (1a) bestimmt wird und das Ultraschallbild in Überlagerung mit dem Bild der Fasern angezeigt wird.

7. Verfahren nach Anspruch 6, wobei das Ultraschallbild durch Bilden von Kanälen ausgehend von den kohärenten Signalen bestimmt werden, die in Schritt (b) bestimmt wird.

8. Verfahren nach einem der vorhergehenden Ansprüche, wobei das abzubildende Medium ein menschliches oder tierisches Gewebe ist.

9. Vorrichtung zum Umsetzen eines Verfahrens zur Abbildung nach einem der vorhergehenden Ansprüche, umfassend ein zweidimensionales Array (2) von Wandlern $T_{ij}$ und Mittel zum Steuern und Verarbeiten (8, 4), die geeignet sind, um:

(a) von dem zweidimensionalen Array (2) von Wandlern $T_{ij}$ in einem Beobachtungsfeld (1a) eines Mediums (1), das Fasern aufweist, eine Anzahl N von einfallenden Ultraschallwellen *I*, die unterschiedliche Wellenfronten aufweisen, zu emittieren, und von den Wandlern $T_{ij}$ für Ultraschallwellen repräsentative Signale $RFraw_{I,ij}(t)$ einzufangen, die jeweils ausgehend von den einfallenden Wellen *I* zurückgestrahlt werden,

(b) ausgehend von den N Sätzen von eingefangenen Signalen $RFraw_{I,ij}(t)$ für eine Anzahl M von fiktiven Fokuspunkten $P_k$ in dem Beobachtungsfeld kohärente Signale $RFcoherent_{k,ij}(t)$ zu bestimmen, die den Signalen entsprechen, die von den Wandlern $T_{ij}$ empfangen worden wären, wenn eine an dem Punkt $P_k$ fokussierte Welle von den Wandlern emittiert worden wäre,

(c) eine Anwesenheit und eine Ausrichtung von Fasern an jedem Punkt $P_k$ zu bestimmen, indem eine räumliche Kohärenz zwischen den kohärenten Signalen $RFcoherent_{k,ij}(t)$ in verschiedenen Richtungen verglichen wird.

**Claims**

1. Method for mapping fibrous media, comprising:

(a) a measurement step during which a two-dimensional array (2) of transducers $T_{ij}$ emits, in a field of view (1a) of a medium (1) comprising fibers, a number N of unfocused incident ultrasonic waves *I* having different wavefronts, and respective signals $RFraw_{I,ij}(t)$ representative of ultrasonic waves reverberated by the medium are captured by the transducers $I_{ij}$ from the incident waves *I*,

(b) a step of synthesizing coherent data during which are determined, from N sets of captured signals $RFraw_{I,ij}(t)$, for a number M of fictitious focal points $P_k$ in the field of view, coherent signals $RFcoherent_{k,ij}(t)$ corresponding to the signals that would have been received by the transducers $I_{ij}$ if a wave focused at point $P_k$ had been emitted by said transducers,

(c) a step of mapping fibers of the medium (1), during which the presence and orientation of fibers at each point $P_k$ are determined by comparing a spatial coherence between coherent signals $RFcoherent_{k,ij}(t)$ in a plurality of directions.

2. Method according to claim 1, wherein, during step (c), an integral of functions of spatial coherence between transducers is determined in a plurality of directions, and the direction of the fibers is determined as being a direction which maximizes said integral.

3. Method according to any preceding claim, wherein the incident ultrasonic waves are plane waves having different propagation directions.

4. Method according to any one of claims 1 to 3, wherein the incident ultrasonic waves are divergent waves.

5. Method according to any preceding claim, wherein an image of the fibers detected in the medium (1) is displayed.

6. Method according to claim 5, wherein an ultrasound image of the field of view is determined (1a) and this ultrasound image is displayed with a superimposed image of the fibers.

7. Method according to claim 6, wherein the ultrasound image is determined by beamforming the coherent signals determined in step (b).

8. Method according to any preceding claim, wherein the medium to be imaged is human or animal tissue.

9. Device for implementing a mapping method according to any one of the preceding claims, comprising a two-dimensional array (2) of transducers $T_{ij}$ as well as control and processing means (8, 4) suitable for:

(a) causing the two-dimensional array (2) of transducers $T_{ij}$ to emit, in a field of view (1a) of a medium (1) comprising fibers, a number N of incident ultrasonic waves $I$ having different wavefronts, and causing the transducers $T_{ij}$ to capture respective signals $RFraw_{I,ij}(t)$ representative of reverberated ultrasonic waves from the incident waves $I$,

(b) determining, from the N sets of captured signals $RFraw_{I,ij}(t)$, for a number M of fictitious focal points $P_k$ in the field of view, coherent signals $RFcoherent_{k,ij}(t)$ corresponding to the signals that would been received by the transducers $T_{ij}$ if a wave focused at point $P_k$ had been emitted by said transducers,

(c) determining the presence and orientation of fibers at each point $P_k$, by comparing spatial coherence between the coherent signals $RFcoherent_{k,ij}(t)$ in a plurality of directions.

## FIG. 1

## FIG. 2

## RÉFÉRENCES CITÉES DANS LA DESCRIPTION

**Documents brevets cités dans la description**

- EP 2101191 A

**Littérature non-brevet citée dans la description**

- **DERODE A. ; FINK M.** Spatial coherence of ultrasonic speckle in composites. *IEEE Trans. Ultrason. Ferroelectr. Freq. Control,* 1993, vol. 40 (6), 666-75

- **MONTALDO et al.** Coherent plane-wave compounding for very high frame rate ultrasonography and transient elastography. *IEEE Trans Ultrason Ferroelectr Freq Control,* Mars 2009, vol. 56 (3), 489-506